(19) Europäisches Patentamt
European Patent Office
Office européen des brevets

(11) Publication number: **0 048 132**
**B1**

(12) **EUROPEAN PATENT SPECIFICATION**

(45) Date of publication of patent specification: **15.08.84**

(21) Application number: **81304140.7**

(22) Date of filing: **10.09.81**

(51) Int. Cl.³: **A 61 K 31/40** //C07D487/04, (C07D487/04, 209/00, 209/00)

(54) **The use of dihydro-1H-pyrrolizine-3,5(2H,6H)-dione as a cognition activator, pharmaceutical compositions containing that compound, and the production of such compositions.**

(30) Priority: **15.09.80 US 186945**
**07.08.81 US 290114**

(43) Date of publication of application:
**24.03.82 Bulletin 82/12**

(45) Publication of the grant of the patent:
**15.08.84 Bulletin 84/33**

(84) Designated Contracting States:
**AT BE CH DE FR GB IT LI LU NL SE**

(56) References cited:

**Keine**

(73) Proprietor: **WARNER-LAMBERT COMPANY**
**201 Tabor Road**
**Morris Plains New Jersey 07950 (US)**

(72) Inventor: **Butler, Donald Eugene**
**1449 Covington Drive**
**Ann Arbor Michigan 48103 (US)**

(74) Representative: **Jones, Michael Raymond et al,**
**HASELTINE LAKE & CO. 28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 048 132 B1

## Description

This invention relates to the use of dihydro-1*H*-pyrrolizine-3,5(2*H*,6*H*)-dione, also known as 3,5-dioxopyrrolizidine and as 1-azabicyclo[3,3,0]octane-2,8-dione, as a cognition activator. The present invention also relates to pharmaceutical compositions containing the compound, and to a process for producing the composition.

The compound dihydro-1*H*-pyrrolizine-3,5(2*H*,6*H*)-dione has the structural formula:—

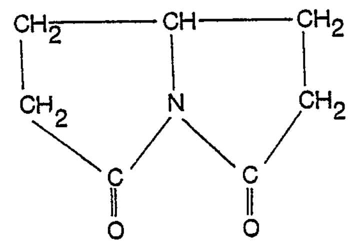

Rodd's Chemistry of Carbon Compounds, 2nd edition, Vol. IVA, (1973), page 480 gives the structural formula of pyrrolizidine as:

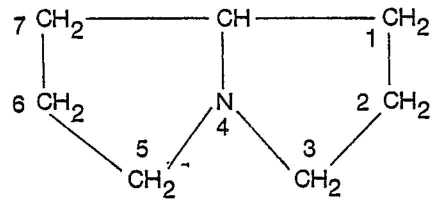

More specifically, the active compound is useful in the treatment of patients suffering from senility and is also useful for enhancing memory and in the treatment of induced amnesia. The active compound may be in the form of a solvate or hydrate. Included in the present invention is the use, as cognition activators, of pharmaceutically acceptable compositions of, and the solvates and hydrates of, the active compound.

The active compound may be produced by modification of the process described by N. J. Leonard et al. in J. Amer. Chem. Soc., *69*, 690—692 (1947). The modified process is described herein. The starting compound may be produced by the process described in United States Patent No. 2,390,918.

Dihydro-1*H*-pyrrolizine-3,5(2*H*,6*H*)-dione may exist in anhydrous form as well as in solvated, included hydrated, forms. In general, the hydrated forms and the solvated forms with pharmaceutically acceptable solvents are equivalent to the anhydrous or unsolvated form for the purposes of the present invention.

In Annalen der Chemie *581*, 225 (1953) is a reference to dihydro-1*H*-pyrrolizine-3,5(2*H*,6*H*)-dione in a very dilute aqueous solution, and the ultraviolet spectrum (on page 226) of the compound shows a log $\varepsilon$ value of approximately 4 at approximately 221 nm (2210 Å), thus indicating that the concentration of the compound is about 1 mg per decilitre of water, a concentration which is too dilute in practical terms for the compound to be administered medicinally.

According to one aspect of the present invention, there is provided a pharmaceutical composition useful for the treatment of senility, enhancing memory or reversing amnesia caused by electroconvulsive shock in a mammal, the composition comprising an effective amount of dihydro-1*H*-pyrrolizine-3,5(2*H*,6*H*)-dione in order to accomplish the treatment in combination with a pharmaceutically acceptable carrier or diluent, with the proviso that, when the diluent is water, the concentration of dihydro-1*H*-pyrrolizine-3,5(2*H*,6*H*)-dione is considerably in excess of 1 mg per decilitre.

When the pharmaceutical composition is in liquid form, preferably the concentration of the active ingredient is at least 20 mg per decilitre.

For producing pharmaceutical compositions from the active compound, inert, pharmaceutically acceptable carriers or diluents can be either solid or liquid. Solid form preparations include powders, tablets, dispersible granules, capsules, cachets and suppositories. A solid carrier can be one or more substances which may also act as diluents, flavouring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to 70 per cent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax or cocoa butter. The term "preparation" is intended

to include the formulation of the active compound with an encapsulating material as carrier, providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized moulds, allowed to cool and thereby to solidify.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colourants, flavours, or stabilizing and thickening agents as desired. Aqueous suspensions suitable for oral use can be made by dispersing the finely divided active component in water with viscous material, i.e. natural or synthetic gums, resins, methylcellulose, sodium carboxymethylcellulose and other well-known suspending agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such as used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e. under refrigeration) in order to retard possible decomposition. The solid form preparations intended to be converted to liquid form may contain, in addition to the active material, flavourants, colourants, stabilizers, buffers, artificial and natural sweeteners, dispersants, thickeners, solubilizing agents and the like. The liquid utilized for preparing the liquid form preparation may be water, isotonic water, ethanol, glycerine, propylene glycol and the like as well as mixtures thereof. Naturally, the liquid utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from 1 mg to 100 mg according to the particular application and the potency of the active ingredient. The compositions can, if desired, also contain other therapeutic agents.

In therapeutic use as cognition activators, the mammalian dosage range for a 70 kg subject is from 1 to 1500 mg/kg of body weight per day or preferably 25 to 750 mg/kg of body weight per day. The dosages, however, may be varied depending upon the requirements of the patient, the severity of the condition being treated and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The effectiveness of the active compound is determined by the test designed to show the compound's ability to reverse amnesia produced by electroconvulsive shock. The test is fully described in United States Patent No. 4,145,347, the only difference being that the test compound in the present case was administered orally.

The following criteria are used in interpreting the percentage of amnesia reversal scores: 40 percent or more (active = A); 25 to 39 percent (borderline = C); and 0 to 24 percent (inactive = N).

The following Table 1 reports the percentage of amnesia reversal of orally administered dihydro-1H-pyrrolizine-3,5 (2H,6H)-dione.

TABLE 1

| Dose mg/kg | 0.63 | 1.25 | 2.50 | 5.00 | 20.00 | 40.00 | 80.00 | 160.00 | 320.00 |
|---|---|---|---|---|---|---|---|---|---|
| % Reversal | 73 | 27 | 25 | 69 | 31 | 92 | 63 | 62 | 85 |
| Rating | A | C | C | A | C | A | A | A | A |

The utility of dihydro-1H-pyrrolizine-3,5(2H,6H)-dione is enchancing memory is demonstrated by the Rodent Delayed Alternation Test.

This test assesses the effects of drugs upon the performance of a short-term memory task in test-sophisticated rodents. The behavioural task employed is delayed spatial alternation using discrete trial procedures in a free operant setting. This task requires the animal to remember from one trial to the next which of two alternative levers was selected on the previous trial. The correct choice in the subsequent trial is always the alternate (or opposite) of that made in the previous trial. By controlling the delay between trials (inter-trial interval, herein abbreviated to ITI), it is possible to measure short-term memory at various times after the to-be-remembered event has occurred.

Preliminary Training

As the delayed alternation taks is somewhat complex, rather extensive training is required before drug testing may begin. Rats are first trained to press a bar (to bar press) for food reinforcement while being maintained on a 22-hour food deprivation schedule. Initially, a single bar press delivers the 45 mg food pellet reward (continuous reinforcement or CRF schedule). The number of presses required is gradually increased until the animal must respond 8 times (FR 8 schedule) to obtain a single pellet. The animals are then trained to alternate their responses between the two available levers. A cue lamp above the correct lever signals the correct manipulation. Once the animal is alternating under the FR-8 requirement the light cue is removed (lamps are lit above both levers) and the animal must remember which is the correct side on the basis of the lever used on the previous trial. When the animals can alternate successfully with modest delays (10—20 s) between trials — generally after 4—6 weeks of training — drug testing begins.

Drug Testing

A drug test consists of two consecutive experimental sessions. The only difference between the two sessions is the oral dosing of the drug on the second test day. The first session is an undrugged control session to compare with the drug performance of the second session.

An experimental session consists of the following procedures: one-half hour prior to being placed in the experimental chamber, the animal is removed from the home cage in the colony room and dosed orally (volume 1 $cm^3$/kg) with the experimental or control solution. (Animals are housed 1—3/cage in the colony and are maintained on 12—12 light/dark cycle with free access to water in the home cage and experimental chamber). The animal is then placed in a retaining cage for the duration of the drug absorption period when the test begins.

Thirty minutes after dosing the animal is placed in the experimental chamber and the test session is initiated by a response from the experimenter. While in the experimental chamber (Skinner box), the animal is presented with a series of trials, each trial separated from the previous by an inter-trial interval (ITI) whose duration is controlled by the experimental apparatus. A trial is signalled by lighted lamps above both levers. Between trials the lamps are dark. The correct lever is always the alternate of that chosen (by meeting the FR-8 requirement) on the previous trial. Incorrect responses — those made on the same lever as that of the previous trial — are counted but do not influence the trial outcome unless the FR-8 requirement is met. If the animal responds correctly 8 times (FR-8), the food reinforcement is delivered and the trial is terminated. If the animal responds 8 times to the incorrect lever, a buzzer is sounded for 1 second and the trial is terminated. A new trial begins after the new ITI value has elapsed. The minimum duration of the ITI is determined by randomly choosing one of four values selected by the experimenter. The randomization procedure has the single constraint that for each block of 20 trials each of the 4 ITI values be selected an equal number of times (5). The test session continues either for a specified period — generally 1—2 hours — or until a certain number of trials or reinforcement has occurred (around 100 trials).

The number of reinforcements that an animal can earn per unit of time is held somewhat constant by rewarding the animal with multiple pellets after long delay intervals. Thus, if an animal must wait an average of 30 seconds to earn one pellet (ITI = 30 seconds), ITI's of 60 seconds and 90 seconds will be rewarded with 2 and 3 pellets respectively, if the animal responds correctly. The shorter intervals are, therefore, rewarded with a single pellet. The longer two delays, ranked according to length, are rewarded with two and three pellets, respectively.

Tests are evaluated on both qualitative and quantitative measures. For the qualitative evaluation, the number of animals showing improvement in performance on the drug day as compared with the control day is computed. The quantitative evaluation is obtained by computing the amount of improvement at each of the delay intervals on drugged vs undrugged days. Since the quantitative evaluation is intended to provide an estimate of the magnitude of the drug's enhancing effect, it is made only for dosages which show activity in the qualitative evaluation.

Thus, particular dosage of drug may receive two ratings: the first related to the reliability of the drug facilitation. The ratings on this measure are listed below:

PERCENTAGE OF ANIMALS SHOWING IMPROVEMENT

| Scores | Ratings |
|---|---|
| 75% and up | A — Active |
| 65% — 74% | C — Borderline |
| 25% — 64% | N — Inactive |
| 24% and less | U — Disruptive |

The dosages showing activity in the qualitative analysis (rating of A or C above) are further analyzed to estimate the extent of the facilitating effects.

This quantitative rating is determined by dividing the difference between drug and control scores at each of the delay intervals by the control score as follows:

$$\% \text{ improvement} = \frac{\text{drug score-control score}}{\text{control score}} \times 100$$

The mean amount of improvement across all delay intervals is then rated according to the following scale:

AMOUNT OF MEMORY ENHANCEMENT

| % Improvement | Rating |
|---|---|
| 20% or more | B — Marked Activity |
| 10—19% | C — Moderate Activity |
| less than 10% | D — Slight Activity |

This test is duplicated to ensure that the results are real. The following Tables 2 and 3 below report typical memory enhancement results caused by dihydro-1H-pyrrolizine-3,5(2*H*,6*H*)-dione.

TABLE 2

| | | | Qualitative Evaluation | | Quantitative Evaluation | | | | | |
|---|---|---|---|---|---|---|---|---|---|---|
| | | | | | | Percent Improvement | | | | |
| | | | | | | | at Delay Time, Sec. | | | |
| Dose | Units* | Rating | No. of Animals | Better Score | Rating | Total | 0–30 | 30–60 | 60–90 | 90–120 |
| 80.00 | M/K | A | 17 | 76% | D | 6% | 5% | 5% | 7% | 4% |
| 40.00 | M/K | N | 13 | 46% | | | | | | |
| 20.00 | M/K | A | 15 | 80% | D | 4% | 3% | 1% | 4% | 8% |
| 10.00 | M/K | C | 15 | 73% | D | 3% | 1% | 1% | 0% | 11% |
| 5.00 | M/K | N | 16 | 63% | D | 0% | 0% | 1% | 2% | 0% |

Qualitative Rating: A – Active (⩾75%); C – Borderline (65%–74%); N – Inactive (25–64%); D – Disruptive (<25%).

Quantitative Rating: B – Marked Activity (⩾20%); C – Moderate Activity (10–19%); D – Slight Activity (<10%).

*M/K = mg/kg of body weight.

TABLE 3

| | | | Qualitative Evaluation | | Quantitative Evaluation | | | | | |
|---|---|---|---|---|---|---|---|---|---|---|
| | | | | | | Percent Improvement | | | | |
| | | | | | | | at Delay Time, Sec. | | | |
| Dose | Units* | Rating | No. of Animals | Better Score | Rating | Total | 0–30 | 30–60 | 60–90 | 90–120 |
| 80.00 | M/K | C | 14 | 71% | D | 6% | 3% | 2% | 3% | 16% |
| 40.00 | M/K | A | 15 | 80% | D | 6% | 0% | 1% | 10% | 8% |
| 20.00 | M/K | A | 16 | 75% | D | 4% | 0% | 1% | 9% | 0% |
| 10.00 | M/K | N | 16 | 38% | | | | | | |
| 5.00 | M/K | N | 15 | 60% | | | | | | |

Qualitative Rating: A – Active (≥75%), C – Borderline (65–74%); N – Inactive (25–64%);

D – Disruptive (<25%)

Quantitative Rating: B – Marked Activity (> or 20%); C – Moderate Activity (10%–19%);

D – Slight Activity (<10%).

*M/K = mg/kg of body weight.

Preparation of dihydro-1*H*-pyrrolizine-3,5(2*H*,6*H*)-dione.

A 7.6 litre (two gallon) 316 stainless steel stirred autoclave was charged with 744 g (3.2 moles) gamma-nitrodimethylpimelate containing 15 g 20% palladium on carbon. Reagent grade methanol (3.78 l) was added and the reactor pressurized to 4.5 x $10^5$ Pascals (50 psig = 3.4 atmospheres gauge) with hydrogen. The stirrer was started, and a smooth uptake of hydrogen was observed over ca five hours, with a maximum temperature of 43°C generated. The reduction mixture was withdrawn after hydrogen uptake ceased, and the catalyst filtered off on a 1.3 cm bed of Super-Cel on a medium porosity sintered glass funnel. The clear, water-white solution was concentrated on a 10 l rotary evaporator at 45°C and 1.6 x $10^2$ Pascals (12 mm). When no more distillate was observed, the temperature of the bath was raised to 100°C to remove the balance of volatiles and promote ring closure of any amino ester present. A yield of 553 g of a viscous dark-yellow oil was obtained.

The product was a mixture of pyrrolidin-2-one-5-(beta-carbomethoxyethyl) and pyrrolidin-2-one-5-propanoic acid, which was dissolved in 600 ml methanol in a 5 l four-necked flask fitted with mechanical stirrer, y-tube, condenser, and thermometer. To the stirred solution was added 400 ml deionized water followed by 296 g (3.70 moles) 50% sodium hydroxide solution. Water (200 ml) was used as a rinse, and the final temperature was 65°C. The resulting tan solution was heated at reflux overnight.

After cooling, the hydrolysis mixture was drawn directly into a 10 l rotary evaporator and the methanol stripped at 45°C and 1.6 x $10^2$ Pascals (12 mm). A solution of 330 ml (3.8 moles) concentrated HCl in 300 ml deionized water was slowly drawn into the rotary evaporator. The balance of volatile material was removed at 60°C and 1.6 x $10^2$ Pascals (12 mm). The thick, pasty residue of pyrrolidin-2-one-5-propanoic acid was slowly treated with 500 ml acetic anhydride (caution) with stirring and scraping to render the material transferable. The resulting slurry was poured into a 5 l, three-necked flask fitted with stirrer and condenser (Drierite protected) along with an additional 1000 ml acetic anhydride. The temperature increased to 45°C during the addition. The tan suspension was heated at 95°C overnight, with stirring.

The dark suspension was then cooled to 60°C and the sodium chloride filtered off through a medium porosity sintered glass funnel. The filter cake was washed with 200 ml hot acetic anhydride and the filtrate stripped of volatiles on a rotary evaporator at 60°C and 1.6 x $10^2$ Pascals (12 mm). Toluene (1 litre) was added to the residue, then 150 ml striped out on a rotary evaporator at 45°C and 1.6 x $10^2$ Pascals (12 mm). The resulting dark solution was ice-bath chilled to 5°C and a crop of solid collected. The filter cake was well pressed to clear all dark supernatant liquid, then washed with 500 ml diethyl ether. The tan solid was dried overnight at 50°C *in vacuo*. The yield of dihydro-1*H*-pyrrolizine-3,5 (2*H*,6*H*)-dione was 211 g, melting at 178°—182°C representing a 48% overall yield including the reduction, hydrolysis, and ring closure steps.

The crude product (211 g) was dissolved in 2.5 l of isopropanol on the steam bath. Fifteen grams Darco G-60 was added and the mixture filtered through a heated sintered glass funnel containing a 1.3 cm layer of Super-gel under a slight vacuum. The filtrate was ice-bath cooled to 5°C and a crop of solid collected. The crystals were washed on the funnel with 200 ml 5°C isopropanol before drying at 50°C *in vacuo*. The yield was 192 g (91% recovery) (43.7% overall) mp 180°—183°C (open capillary).

Production of Dihydro-1H-pyrrolizine-3,5(2H,6H)-dione.

A solution of 136 of 2-nitropimelic acid dimethyl ester in 500 ml of methanol was hydrogenated at approximately 2.6 x $10^7$ Pascals (3780 psi) using 15 g of Raney nickel as catalyst. The resulting slurry was filtered to remove the catalyst and the filtrate was concentrated at reduced pressure to yield crude 5-oxo-pyrrolidin-2-yl-propanoic acid methyl ester was dissolved in 100 ml of methanol and 100 ml of water and was treated with 94 g of 50% sodium hydroxide solution. The reaction mixture was stirred and was heated to 98°C with distillation of methanol. The solution was cooled, neutralized with 151 ml of concentrated hydrochloric acid and concentrated at reduced pressure. The residue which contained 5-oxo-pyrrolidin-2-yl-propanoic acid was heated at 98—100°C for 24 hours with 200 ml of acetic anhydride. The sodium chloride was removed by filtration after the acetic anhydride reaction. The filtrate was then concentrated at reduced pressure and 200 ml of toluene were added and concentration was repeated. Dihydro-1H-pyrrolizine-3,5(2H,6H)-dione crystallised and was isolated by filtration. The product was best purified by sublimation at 100°C and 1.3 x $10^2$ Pascals (0.1 mm Hg) or by recrystallization from ethanol, and it then had a melting point of 179—182°C.

The present invention is further illustrated by the following Examples which refer to the use of tablets and capsules containing 1.0, 2.5, 25, 50 mg respectively of active compound and to the use of a parenteral formulation.

Example 1

| Ingredient | Quantity |
|---|---|
| dihydro-1*H*-pyrrolizine-3,5(2*H*,6*H*)-dione | 150 g |
| lactose | 1124 g |
| corn starch | 39 g |
| hydroxypropyl cellulose | 30 g |
| magnesium stearate | 7 g |
| ethanol-water 50:50 | qs |

The dihydro-1*H*-pyrrolizine-3,5(2*H*,6*H*)-dione, lactose and hydroxypropyl cellulose were blended and granulated with 50:50 ethanol-water. The wet granulation was screened, dried, and rescreened. The resulting dried granulation was blended with the magnesium stearate and corn starch, and the resulting mixture was compressed into 225 mg tablets using a 8.73 mm (11/32 inch) standard concave punch. The yield equalled approximately 6000 tablets each containing 25.0 mg of dihydro-1*H*-pyrrolizine-3,5(2*H*,6*H*)-dione.

Example 2

| Ingredient | Quantity |
|---|---|
| dihydro-1*H*-pyrrolizine-3,5(2*H*,6*H*)-dione | 15 g |
| lactose | 1259 g |
| corn starch | 39 g |
| hydroxypropyl cellulose | 30 g |
| magnesium stearate | 7 g |
| ethanol-water 50:50 | qs |

The dihydro-1*H*-pyrrolizine-3,5(2*H*,6*H*)-dione, lactose and hydroxypropyl cellulose were blended and granulated with 50:50 ethanol-water. The wet granulation was screened, dried and rescreened. The resulting dried granulation was blended with the magnesium stearate and corn starch, and the resulting mixture was compressed into 225 mg tablets using 8.73 mm (11/32 inch) standard concave punch. The yield equalled approximately 6000 tablets each containing 2.5 mg of dihydro-1*H*-pyrrolizine-3,5(2*H*,6*H*)-dione.

Example 3

| Ingredient | Quantity |
|---|---|
| dihydro-1*H*-pyrrolizine-3,5(2*H*,6*H*)-dione | 6 g |
| lactose | 1268 g |
| corn starch | 39 g |
| hydroxypropyl cellulose | 30 g |
| magnesium stearate | 7 g |
| ethanol-water 50:50 | qs |

The dihydro-1*H*-pyrrolizine-3,5(2*H*,6*H*)-dione, lactose and hydroxypropyl cellulose were blended and granulated with 50:50 ethanol-water. The wet granulation was screened, dried, and rescreened.

The resulting dried granulation was blended with the magnesium stearate and corn starch, and the resulting mixture was compressed into 225 mg tablets using a 8.73 mm (11/32 inch) standard concave punch. The yield equalled approximately 6000 tablets each containing 1.0 mg of dihydro-1*H*-pyrrolizine-3,5(2*H*,6*H*)-dione.

Example 4

| Ingredient | Quantity |
|---|---|
| dihydro-1*H*-pyrrolizine-3,5(2*H*,6*H*)-dione | 300 g |
| lactose | 974 g |
| corn starch | 39 g |
| hydroxypropyl cellulose | 30 g |
| magnesium stearate | 7 g |
| ethanol-water 50:50 | qs |

The dihydro-1H-pyrrolizine-3,5(2H,6H)-dione, lactose and hydroxypropyl cellulose were blended and granulated with 50:50 ethanol-water. The wet granulation was screened, dried, and rescreened. The resulting dried granulation was blended with the magnesium stearate and corn starch and the resulting mixture was compressed into 225 mg tablets using a 8.73 mm (11/32 inch) standard concave punch. The yield equalled approximately 6000 tablets each containing 50.0 mg of dihydro-1*H*-pyrrolizine-3,5 (2*H*,6*H*)-dione.

Example 5

| Ingredient | Quantity |
|---|---|
| dihydro-1*H*-pyrrolizine-3,5(2*H*,6*H*)-dione | 250 g |
| lactose | 1723 g |
| magnesium stearate | 27 g |

The mixture was blended and filled into No. 4 hard gelatin capsules, filling each capsule with 200 mg of the powder mixture. The yield equalled approximately 10,000 capsules each containing 25.0 mg of dihydro-1*H*-pyrrolizine-3,5(2*H*,6*H*)-dione.

Example 6

| Ingredient | Quantity |
|---|---|
| dihydro-1*H*-pyrrolizine-3,5(2*H*,6*H*)-dione | 25 g |
| lactose | 1948 g |
| magnesium stearate | 27 g |

The mixture was blended and filled into No. 4 hard gelatin capsules, filling each capsule with 200 mg of the powder mixture. The yield equalled approximately 10,000 capsules each containing 2.5 mg of dihydro-1*H*-pyrrolizine-3,5(2*H*,6*H*)-dione.

Example 7

| Ingredient | Quantity |
|---|---|
| dihydro-1*H*-pyrrolizine-3,5(2*H*,6*H*)-dione | 10 g |
| lactose | 1963 g |
| magnesium stearate | 27 g |

The mixture was blended and filled into No. 4 hard gelatin capsules, filling each capsule with 200 mg of the powder mixture. The yield equalled approximately 10,000 capsules each containing 1.0 mg of dihydro-1*H*-pyrrolizine-3,5(2*H*,6*H*)-dione.

Example 8

| Ingredient | Quantity |
|---|---|
| dihydro-1*H*-pyrrolizine-3,5(2*H*,6*H*)-dione | 500 g |
| lactose | 1473 g |
| magnesium stearate | 27 g |

The mixture was blended and filled into No. 4 hard gelatin capsules, filling each capsule with 200 mg of the powder mixture. The yield equalled approximately 10,000 capsules each containing 50.0 mg of dihydro-1*H*-pyrrolizine-3,5(2*H*,6*H*)-dione.

Example 9

| Ingredient | Quantity |
|---|---|
| dihydro-1*H*-pyrrolizine-3,5(2*H*,6*H*)-dione | 1.0 mg |
| phemerol chloride recrystallized | 0.1 mg |
| water for injection USP | qs and 1.0 ml |

The dihydro-1H-pyrrolizine-3,5(2H,6H)-dione was mixed with about two thirds of the required volume of water for injection USP followed by the addition of sufficient water for injection to reach the desired volume. After mixing, the solution was sterilized by membrane filtration (a 0.22 Millipore filter membrane represented a suitable filter). The desired quantity of the above-prepared solution was introduced into approximate size multiple dose vials suitable for injection preparations and stopped with gum rubber or suitable rubber closures and sealed with aluminium ferrules. The preparation may also be filled into suitable size single dose glass ampoules and sealed.

Using the above procedure, solutions containing 1.0, 2.5, 5.0 or 10.0 mg/ml of dihydro-1*H*-pyrrolizine-3,5(2*H*,6*H*)-dione were prepared.

Example 10

Suppository formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 500 g |
| Carrier (mixed fatty acid glycerides) | 1,500 g |

Fifteen hundred grams of the carrier were heated until melted. Then 500 grams of dihydro-1*H*-pyrrolizine-3,5 (2*H*,6*H*)-dione were added and mixed until the mixture was uniform.

The molten mixture was poured into standard two gram moulds and moulded at 34°C. The yield equalled approximately 1000 2 gram-suppositories each containing 500 mg of dihydro-1*H*-pyrrolizine-3,5(2*H*,6*H*)-dione.

Example 11

Suppository formulation of dihydro-1*H*-pyrrolizine-3,5 (2*H*,6*H*)-dione.

| Ingredient | Quantity |
|---|---|
| dihydro-1*H*-pyrrolizine-3,5(2*H*,6*H*)-dione | 30 g |
| Carrier (mixed fatty acid glycerides) | 1,970 g |

One thousand nine hundred and seventy grams of the carrier were heated until melted. Then 30 grams of the dihydro-1*H*-pyrrolizine-3,5(2*H*,6*H*)-dione were added and mixed until the mixture was uniform. The molten mixture was poured into standard two gram moulds and moulded at 34°C. The

yield equalled approximately 1000 2 gram-suppositories each containing 30 mg of dihydro-1*H*-pyrrolizine-3,5(2*H*,6*H*)-dione.

Example 12

Suspension formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 200 g |
| sodium saccharin | 50 g |
| trihydroxystearin | 100 g |
| propylparaben | 10 g |
| imitation cherry flavour | 20 g |
| vehicle (caprylic/capric triglycerides) | q.s. to 1000 ml |

The 10 grams of propylparaben were added to 400 grams of the vehicle and the mixture was heated to 55°C until solution is complete. The 100 grams of trihydroxystearin were added and the mixture was homogenized until the temperature reached 45°C. The mixture was cooled to room temperature and 200 grams of dihydro-1*H*-pyrrolizine-3,5(2*H*,6*H*)-dione, 20 grams of imitation cherry flavour and 50 grams of sodium saccharin were added. The suspension was diluted to 1000 ml with the vehicle. This yielded a suspension containing 1 gram of dihydro-1*H*-pyrrolizine-3,5(2*H*,6*H*)-dione per 5 ml of suspension.

Example 13

Suspension formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 20 g |
| sodium saccharin | 25 g |
| trihydroxystearin | 100 g |
| propylparaben | 10 g |
| imitation cherry flavour | 20 g |
| vehicle (caprylic/capric triglycerides) | q.s. to 1000 ml |

The 10 grams of propylparaben were added to 400 grams of the vehicle and the mixture was heated to 500°C until the solution was complete. The 100 grams of trihydroxystearin were added and the mixture was homogenized until the temperature reached 45°C. The mixture was cooled to room temperature and 20.0 grams of dihydro-1*H*-pyrrolizine-3,5(2*H*,6*H*)-dione, 25 grams of sodium saccharin and 20 grams of imitation cherry flavour were added. The suspension was diluted to 1000 ml with the vehicle. The suspension was diluted to 1000 ml with the vehicle. This yielded a suspension containing 100 mg of dihydro-1*H*-pyrrolizine-3,5(2*H*,6*H*)-dione per 5 ml of suspension.

Example 14

Suspension formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 50 g |
| sodium saccharin | 50 g |
| trihydroxystearin | 50 g |
| propylparaben | 10 g |
| imitation cherry flavour | 20 g |
| vehicle (caprylic/capric triglycerides) | q.s. to 1000 ml |

The 10 grams of propylparaben were added to 400 g of the vehicle and the mixture was heated to 55°C until the solution was complete. The 50 grams of trihydroxystearin were added and the mixture was homogenized until the temperature reached 45°C. The mixture was cooled to room temperature and 50 grams of dihydro-1*H*-pyrrolizine-3,5 (2*H*,6*H*)-dione, 50 grams of sodium saccharin and 20 grams of imitation cherry flavour were added. The suspension was diluted to 1000 ml with the vehicle. This yielded a suspension containing 250 mg of dihydro-1*H*-pyrrolizine-3,5(2*H*,6*H*)-dione per 5 ml of suspension.

Example 15

Syrup for reconstitution formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 100 g |
| sugar granulated (bottlers grade) | 400 g |
| artificial peppermint flavour water soluble spray dried | 10 g |
| water | q.s. to 1000 ml at time of dispensing |

There were dry blended the 100 grams of dihydro-1*H*-pyrrolizine-3,5(2*H*,6*H*)-dione, 400 grams of granulated sugar (bottlers grade) and 10 grams of artificial peppermint flavour. Water was added q.s. to 1000 ml at time of dispensing, and shaken until dissolution. Refrigerated storage and use within one week was recommended. This syrup contained 500 mg of dihydro-1*H*-pyrrolizine-3,5 (2*H*,6*H*)-dione per 5 ml of syrup.

Example 16

Syrup for reconstitution formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 50 g |
| sugar granulated (bottlers grade) | 400 g |
| artificial peppermint flavour water soluble spray dried | 10 g |
| water | q.s. to 1000 ml at time of dispensing |

There were dry blended 50 grams of dihydro-1*H*-pyrrolizine-3,5(2*H*,6*H*)-dione, 400 grams of granulated sugar (bottlers grade) and 10 grams of artificial peppermint flavour. Water was added q.s. to 1000 ml at the time of dispensing, and shaken until dissolution. Refrigerated storage and use within one week was recommended. This syrup contained 250.0 mg of dihydro-1*H*-pyrrolizine-3,5(2*H*,6*H*)-dione per 5 ml of syrup.

Example 17

Syrup for reconstitution formulation of dihydro-1*H*-pyrrolizine-3,5(2*H*,6*H*)-dione.

| Ingredient | Quantity |
|---|---|
| dihydro-1*H*-pyrrolizine-3,5(2*H*,6*H*)-dione | 25 g |
| sugar granulated (bottlers grade) | 400 g |
| artificial peppermint flavour water soluble spray dried | 10 g |
| water | q.s. to 1000 ml at time of dispensing |

There were dry blended 25 grams of dihydro-1*H*-pyrrolizine-3,5(2*H*,6*H*)-dione, 400 grams of granulated sugar (bottlers grade) and 10 grams of artificial peppermint flavour. Water was added q.s. to 1000 ml at the time of dispensing, and shaken until dissolution. Refrigerated storage and use within one week was recommended. This syrup contained 125 mg of dihydro-1*H*-pyrrolizine-3,5 (2*H*,6*H*)-dione per 5 ml of syrup.

**Claims for the Contracting States: BE CH DE FR GB IT LI LU NL SE**

1. A pharmaceutical composition useful for the treatment of senility, enhancing memory or reversing amnesia caused by electroconvulsive shock in a mammal, the composition comprising an effective amount of dihydro-1*H*-pyrrolizine-3,5(2*H*,6*H*)-dione in order to accomplish the treatment in combination with a pharmaceutically acceptable carrier or diluent, with the proviso that, when the diluent is water, the concentration of dihydro-1*H*-pyrrolizine-3,5(2H,6*H*)-dione is considerably in excess of 1 mg per decilitre.
2. A composition according to Claim 1, which is in the form of a solid.
3. A composition according to Claim 1, which is in the form of a liquid.
4. A composition according to Claim 3, wherein the liquid form composition is a solution, suspension or emulsion.
5. A composition according to Claim 3 or 4, wherein the concentration of dihydro-1*H*-pyrrolizine-3,5(2*H*,6*H*)-dione is at least 20 mg per decilitre.
6. A composition according to any preceding claim, which is in unit dosage form.
7. Dihydro-1*H*-pyrrolizine-3,5(2*H*,6*H*)-dione, for use in the treatment of senility, or in enhancing memory or in reversing amnesia caused by electroconvulsive shock in a mammal.

**Claims for the Contracting State: AT**

1. A process for producing a pharmaceutical composition useful for the treatment of senility, enhancing memory or reversing amnesia caused by electroconvulsive shock in a mammal, which process comprises incorporating an effective amount of dihydro-1*H*-pyrrolizine-3,5(2*H*,6*H*)-dione in order to accomplish the treatment, in a pharmaceutically acceptable carrier or diluent, with the proviso that, when the diluent is water, the concentration of dihydro-1*H*-pyrrolizine-3,5(2*H*,6*H*)-dione is considerably in excess of 1 mg per decilitre.
2. A process according to Claim 1, wherein the carrier is in the form of a solid.
3. A process according to Claim 1, wherein the diluent is in the form of a liquid.
4. A process according to Claim 2, wherein the composition in solid form is converted to a liquid form composition before use.
5. A process according to Claim 3 or 4, wherein the concentration of dihydro-1*H*-pyrrolizine-3,5(2*H*,6*H*)-dione is at least 20 mg per decilitre.
6. Dihydro-1*H*-pyrrolizine-3,5(2*H*,6*H*)-dione or a pharmaceutical composition incorporating that compound, for use in the treatment of senility, or in enhancing memory or in reversing amnesia caused by electroconvulsive shock in a mammal.

**Patentansprüche für die Vertragsstaaten: BE CH DE FR GB IT LI LU NL SE**

1. Pharmazeutische Zusammensetzung, welche sich für die Behandlung der Senilität, Verbesserung des Gedächtnisses oder zum Rückgängigmachen von durch Elektroschock in einem Säuger hervorgerufener Amnesie, welche Zusammensetzung dadurch gekennzeichnet ist, daß sie eine für die Durchführung der Behandlung wirksame Menge an Dihydro-1H-pyrrolizin-3,5(2H,6H)-dion in Kombination mit einem pharmazeutisch akzeptablen Träger oder Verdünnungsmittel umfaßt, mit der Maßgabe, daß, wenn das Verdünnungsmittel Wasser ist, die Konzentration des Dihydro-1H-pyrrolizin-3,5(2H,6H)dion beträchtlich über 1 mg pro Deziliter liegt.
2. Zusammensetzung nach Anspruch 1, dadurch gekennzeichnet, daß sie in Form eines Feststoffs vorliegt.
3. Zusammensetzung nach Anspruch 1, dadurch gekennzeichnet, daß sie in Form einer Flüssigkeit vorliegt.
4. Zusammensetzung nach Anspruch 3, dadurch gekennzeichnet, daß die Zusammensetzung in flüssiger Form eine Lösung, Suspension oder Emulsion ist.
5. Zusammensetzung nach Anspruch 3 oder 4, dadurch gekennzeichnet, daß die Konzentration von Dihydro-1H-pyrrolizin-3,5-(2H,6H)-dion zumindest 20 mg pro Deziliter beträgt.
6. Zusammensetzung nach einem der vorhergehenden Ansprüche, dadurch gekennzeichnet, daß sie in Einheitsdosisform vorliegt.
7. Dihydro-1H-pyrrolizin-3,5(2H,6H)-dion zur Verwendung bei der Behandlung von Senilität, oder zur Verbesserung des Gedächtnisses oder zum Rückgängigmachen von durch Elektroschock in einem Säuger hervorgerufener Amnesie.

**Patentansprüche für den Vertragsstaat: AT**

1. Verfahren zur Herstellung einer für die Behandlung von Senilität, für die Verbesserung des Gedächtnisses oder zum Rückgängigmachen von durch Elektroschock in einem Säuger hervorgerufener Amnesie geeigneten Zusammensetzung, welches Verfahren dadurch gekennzeichnet ist, daß eine zur Durchführung der Behandlung wirksame Menge an Dihydro-1H-pyrrolizin-3,5(2H,6H)-dion in einen pharmazeutisch akzeptablen Träger oder ein Verdünnungsmittel inkorporiert wird, mit der Maßgabe, daß, wenn das Verdünnungsmittel Wasser ist, die Konzentration des Dihydro-1H-pyrrolizin-3,5(2H,6H)-dions beträchtlich über 1 mg pro Deziliter liegt.

2. Verfahren nach Anspruch 1, dadurch gekennzeichnet, daß der Träger in Form eines Feststoffs vorliegt.

3. Verfahren nach Anspruch 1, dadurch gekennzeichnet, daß das Verdünnungsmittel in Form einer Flüssigkeit vorliegt.

4. Verfahren nach Anspruch 2, dadurch gekennzeichnet, daß die Zusammensetzung in fester Form vor ihrer Verwendung in eine Zusammensetzung in flüssiger Form umgewandelt wird.

5. Verfahren nach Anspruch 3 oder 4, dadurch gekennzeichnet, daß die Konzentration des Dihydro-1H-pyrrolizin-3,5(2H,6H)-dions zumindest 20 mg pro Deziliter beträgt.

6. Verwendung von Dihydro-1H-pyrrolizin-3,5(2H,6H)-dion oder einer diese Verbindung inkorporierenden pharmazeutischen Zusammensetzung zur Behandlung der Senilität, Verbesserung des Gedächtnisses oder zum Rückgängigmachen von durch Elektroschock in einem Säuger hervorgerufener Amnesie.

**Revendications pour les Etats contractants: BE CH DE FR GB IT LU LI NL SE**

1. Composition pharmaceutique utile pour le traitement de la sénilité, l'amélioration de la mémoire ou la suppression de l'amnésie causée par un choc électroconvulsif chez le mammifère, composition contenant une quantité efficace de dihydro-1H-pyrrolizine-3,5(2H,6H)dione en vue d'accomplir le traitement en combinaison avec un véhicule ou dilant pharmaceutiquement acceptable sous réserve que lorsque le diluant est l'eau, la concentration de la dihydro-1H-pyrrolizine-3,5-(2H,6H)-dione soit considérablement supérieure à 1 mg-dl.

2. Composition selon la revendication 1, caractérisée en ce qu'elle se trouve sous la forme d'un solide.

3. Composition selon la revendication 1, caractérisé en ce qu'elle se trouve sous la forme d'un liquide.

4. Composition selon la revendication 3, caractérisé en ce que la composition de forme liquide est une solution, une suspension ou une émulsion.

5. Composition selon la revendication 3 ou 4, caractérisée en ce que la concentration en dihydro-1H-pyrrolizine-3,5(2H,6H)-dione est d'au moins 20 mg/dl.

6. Composition selon l'une quelconque des revendications précédentes, caractérisée en ce qu'elle se trouve sous forme de dosage unitaire.

7. Dihydro-1H-pyrrolizine-3,5(2H,6H)-dione utilisable dans le traitement de la sénilité ou dans l'amélioration de la mémoire ou dans la suppression de l'amnésie causée par un choc électroconvulsif chez un mammifère.

**Revendications pour l'Etat contractant: AT**

1. Procédé de production d'une composition pharmaceutique utile pour le traitement de la sénilité, l'amélioration de la mémoire ou la suppression de l'amnésie causée par un choc électroconvulsif chez un mammifère, ce procédé étant caractérisé en ce que l'on incorpore une quantité efficace de dihydro-1H-pyrrolizine-3,5(2H,6H)-dione en vue d'accomplir le traitement dans un véhicule ou diluant pharmaceutiquement acceptable, sous réserve que lorsque le diluant est l'eau, la concentration en dihydro-1H-pyrrolizine-3,5(2H,6H)-dione soit considérablement supérieure à 1 mg/dl.

2. Procédé selon la revendication 1, caractérisé en ce que le véhicule est sous la forme d'un solide.

3. Procédé selon la revendication 1, caractérisé en ce que le diluant est sous la forme d'un liquide. transformée en une composition de forme liquide avant usage.

4. Procédé selon la revendication 2, caractérisé en ce que la composition de forme solide est transformée en une composition de forme liquide avant usage.

5. Procédé selon la revendication 3 ou 4, caractérisé en ce que la concentration de la dihydro-1H-pyrrolizine-3,5(2H,6H)-dione est d'au moins 20 mg/dl.

6. Dihydro-1H-pyrrolizine-3,5(2H,6H)-dione ou composition pharmaceutique comprenant ce composé utilisable dans le traitement de la sénilité ou dans l'amélioration de la mémoire ou dans la suppression de l'amnésie causée par un choc électroconvulsif chez un mammifère.